# EUROPÄISCHE PATENTSCHRIFT

(11) **EP 1 969 112 B1**
(45) Veröffentlichungstag und Bekanntmachung des Hinweises auf die Patenterteilung: **29.06.2011**
(21) Anmeldenummer: 06829423.0
(22) Anmeldetag: 08.12.2006
(51) Int. Cl.: C11D 3/39, C11D 3/16, C11D 3/28, B01J 31/18, C07D 471/12, C07F 1/08, C07F 11/00, C07F 13/00, C07F 15/00

(54) **REINIGUNGSMITTEL MIT BLEICHKATALYTISCH AKTIVEN KOMPLEXEN**
CLEANING AGENT COMPRISING COMPLEXES WITH BLEACH CATALYTIC ACTIVITY
DÉTERGENT CONTENANT DES COMPLEXES AYANT UNE ACTIVITÉ CATALYTIQUE DE BLANCHIMENT

(30) Priorität: 29.12.2005 DE 102005063059
(43) Veröffentlichungstag der Anmeldung: 17.09.2008
(73) Patentinhaber: Henkel AG & Co. KGaA, 40589 Düsseldorf (DE)
(72) Erfinder: GENTSCHEV, Pavel, 40599 Düsseldorf (DE); KESSLER, Arnd, 40789 Monheim-Baumberg (DE); JEKEL, Maren, 47877 Willich (DE); ZIPFEL, Johannes, 40593 Düsseldorf (DE); NITSCH, Christian, 40591 Düsseldorf (DE); DAHLMANN, Doris, 40589 Düsseldorf (DE); DÖRING, Steve, 40822 Mettmann (DE)
(86) Internationale Anmeldenummer: PCT/EP2006/011817
(87) Internationale Veröffentlichungsnummer: WO 2007/079858

(56) Entgegenhaltungen:
- EP-A2- 1 531 193
- WO-A-99/65905
- WO-A-2005/061688
- VEDERNIKOV A N ET AL: "INFLUENCE OF THE (2.1.1)-(2,6)-PYRIDINOPHANE MACROCYCLE RING SIZE CONSTRAINT ON THE STRUCTURE AND REACTIVITY OF COPPER COMPLEXES" INORGANIC CHEMISTRY, AMERICAN CHEMICAL SOCIETY, EASTON, US, Bd. 43, Nr. 14, 25. Mai 2004 (2004-05-25), Seiten 4300-4305, XP008074923 ISSN: 0020-1669
- VEDERNIKOV A N ET AL: "DESIGN AND SYNTHESIS OF TRIDENTATE FACIALLY CHELATING LIGANDS OF THE (2.N.1)-(2,6)-PYRIDINOPHANE FAMILY" JOURNAL OF ORGANIC CHEMISTRY, AMERICAN CHEMICAL SOCIETY. EASTON, US, Bd. 68, Nr. 12, 14. Mai 2003 (2003-05-14), Seiten 4806-4814, XP008074906 ISSN: 0022-3263
- JENNY W ET AL: "(2.2....)(2,6) PYRIDINOPHANE" CHIMIA, AARAU, CH, Bd. 23, Nr. 4, April 1969 (1969-04), Seiten 158-160, XP008074880 ISSN: 0009-4293

## Beschreibung

Die vorliegende Erfindung betrifft die Verwendung von bestimmten Übergangsmetallkomplexen als katalytisch wirksame Aktivatoren für insbesondere anorganische Persauerstoffverbindungen zum Bleichen von gefärbten Anschmutzungen an harten Oberflächen und Reinigungsmittel für harte Oberflächen, die derartige Katalysatoren enthalten.

Anorganische Persauerstoffverbindungen, insbesondere Wasserstoffperoxid und feste Persauerstoffverbindungen, die sich in Wasser unter Freisetzung von Wasserstoffperoxid lösen, wie Natriumperborat und Natriumcarbonat-Perhydrat, werden seit langem als Oxidationsmittel zu Desinfektions- und Bleichzwecken verwendet. Die Oxidationswirkung dieser Substanzen hängt in verdünnten Lösungen stark von der Temperatur ab; so erzielt man beispielsweise mit H₂O₂ oder Perborat in alkalischen Bleichflotten erst bei Temperaturen oberhalb von etwa 80 °C eine ausreichend schnelle Bleiche verschmutzter Textilien. Bei niedrigeren Temperaturen kann die Oxidationswirkung der anorganischen Persauerstoffverbindungen durch Zusatz sogenannter Bleichaktivatoren verbessert werden, für die zahlreiche Vorschläge, vor allem aus den Stoffklassen der N- oder O-Acylverbindungen, beispielsweise mehrfach acylierte Alkylendiamine, insbesondere Tetraacetylethylendiamin, acylierte Glykolurile, insbesondere Tetraacetylglykoluril, N-acylierte Hydantoine, Hydrazide, Triazole, Hydrotriazine, Urazole, Diketopiperazine, Sulfurylamide und Cyanurate, außerdem Carbonsäureanhydride, insbesondere Phthalsäureanhydrid, Carbonsäureester, insbesondere Natrium-nonanoyloxy-benzolsulfonat, Natrium-isononanoyloxybenzolsulfonat und acylierte Zuckerderivate, wie Pentaacetylglukose, in der Literatur bekannt geworden sind. Durch Zusatz dieser Substanzen kann die Bleichwirkung wäßriger Peroxidflotten so weit gesteigert werden, daß bereits bei Temperaturen um 60 °C im Wesentlichen die gleichen Wirkungen wie mit der Peroxidflotte allein bei 95 °C eintreten.

Auch diese Temperaturen sind für das manuelle Reinigen harter Oberflächen, beispielweise von Geschirr, noch zu hoch und werden normalerweise auch in maschinellen Geschirrspülverfahren nicht immer erreicht. Im Bemühen um energiesparende Verfahren zum maschinellen Reinigen von Geschirr gewinnen in den letzten Jahren Anwendungstemperaturen von unterhalb 60 °C, insbesondere unterhalb 50 °C bis hinunter zur Kaltwassertemperatur an Bedeutung.

Bei diesen niedrigen Temperaturen läßt die Wirkung der bisher bekannten Aktivatorverbindungen in der Regel erkennbar nach, besonders bei schwer bleichbaren Anschmutzungen wie beispielsweise Teerückständen auf Porzellan oder Glas. Es hat deshalb nicht an Bestrebungen gefehlt, für diesen Temperaturbereich wirksamere Aktivatoren zu entwickeln, ohne daß bis heute ein überzeugender Erfolg zu verzeichnen gewesen wäre. Ein Ansatzpunkt dazu könnte sich durch den Einsatz von Ubergangsmetallsalzen und -komplexen als sogenannte Bleichkatalysatoren ergeben. Aus der europäischen Patentanmeldung EP 630 964 sind zum Beispiel bestimmte Mangankomplexe vom Salen-Typ bekannt, welche wie dort angegeben keinen ausgeprägten Effekt hinsichtlich einer Bleichverstärkung von Persauerstoffverbindungen haben und nur die Bleiche von in Waschlaugen befindlichem, das heißt bereits von der zu reinigenden Textiloberfläche abgelöstem Schmutz oder Farbstoff bewirken können.

Überraschenderweise wurde nun gefunden, daß Übergangsmetallkomplexe mit einem makrozyklischen Liganden, der 3 Pyridin-Gruppierungen aufweist, eine deutliche bleichkatalysierende Wirkung auf gefärbte Anschmutzungen haben, die sich an harten Oberflächen befinden.

Gegenstand der Erfindung ist demgemäß die Verwendung von Übergangsmetall-Komplexen der Formel (I),

[MLₐX_{b}] cY (I)

in der
- M: für Mangan, Eisen, Cobalt, Kupfer, Ruthenium oder Molybdän,
- L: für den makrozyklischen Liganden 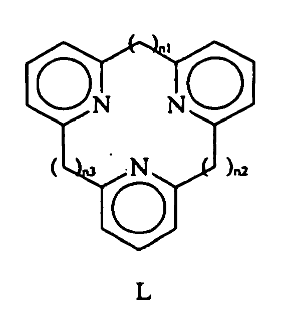 in dem n1, n2 und n3 unabhängig voneinander 1 oder 2 sind,
- X: für einen Neutral- oder Anionliganden,
- Y: für ein nicht komplexgebundenes Anion,
- a: für 1 oder 2 steht und
- b und c: unabhängig voneinander für Zahlen von 0 bis 6 stehen mit der Maßgabe, daß ihre Summe so zu wählen ist, daß sich in Abhängigkeit von der Ladung des Zentralatoms M und der Ladungen von X und Y Neutralität der Verbindung ergibt,
als Aktivatoren für insbesondere anorganische Persauerstoffverbindungen in Reinigungslösungen für harte Oberflächen, insbesondere für Geschirr.

Bevorzugtes Übergangsmetall (M in Formel I) ist Mangan.

In den Verbindungen L können die Pyridinringe mit drei Ethyleneinheiten verbrückt sein (n1 = n2 = n3 = 2), vorzugsweise liegen dort zwei Ethyleneinheiten und eine Methylenheit (n1 = n2 = 2, n3 = 1) oder eine Ethyleneinheit und zwei Methylenheiten (n1 = 2, n2 = n3 = 1) und besonders bevorzugt drei Methyleneinheiten (n1 = n2 = n3 = 1) vor.

Die Herstellung des makrozyklischen Liganden in Formel (I) mit n1 = n2 = n3 = 1 kann durch die folgenden Schritte erfolgen, welche für andere Werte der Indizes n1, n2 und/oder n3 analog angewendet werden können:

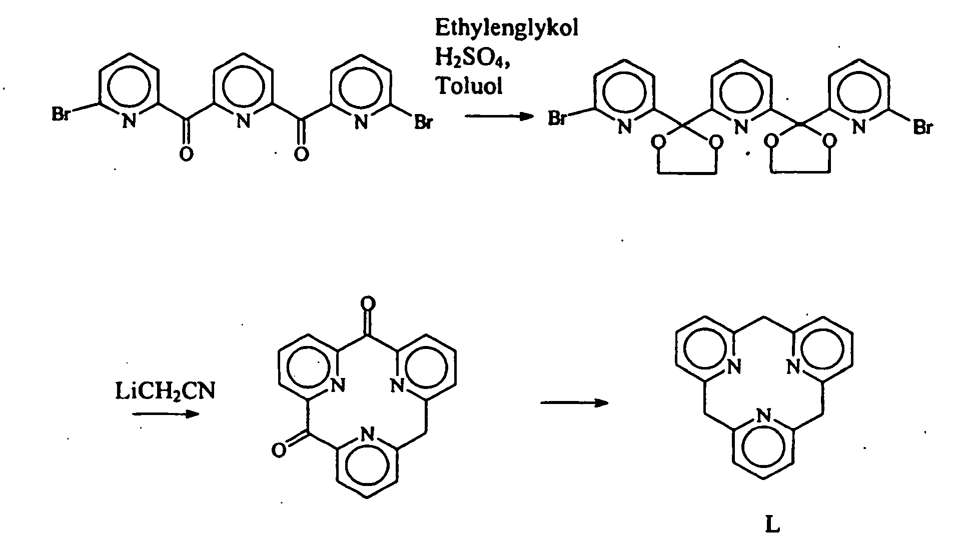

Neutralliganden X in den Komplexverbindungen der Formel (I) können zum Beispiel Wasser oder Ammoniak sein. Ein gegebenenfalls zusammen mit nicht komplex gebundenen Anionen Y ladungsausgleichender Anionligand X in den Verbindungen der Formel (I) kann, ebenso wie das Anion Y, ein- oder mehrwertig sein. Vorzugsweise handelt es sich bei X und Y um ein Halogenid, insbesondere Chlorid, Hydroxid, Hexafluorophosphat, Perchlorat, ein Oxo-, Peroxo- oder Hydroperoxo-Anion oder um das Anion einer Carbonsäure, wie Formiat, Acetat, Benzoat oder Citrat. Anionen Y und/oder Anionliganden X sind in einer solchen Anzahl (c beziehungsweise b in Formel I) vorhanden, dass die Verbindung nach Formel (I) insgesamt keine Ladung aufweist.

Ein weiterer Gegenstand der Erfindung ist die entsprechende Verwendung der makrozyklischen Verbindung L in Gegenwart eines Salzes von Mangan, Eisen, Cobalt, Kupfer, Ruthenium oder Molybdän zur Verstärkung der Bleichleistung von insbesondere anorganischen Persauerstoffverbindungen in Reinigungslösungen für harte Oberflächen. Bei Anwesenheit von Wasser bildet sich aus dem Metallsalz und der Verbindung L vermutlich ein Komplex der Formel (I).

Weiterhin betrifft die Erfindung Reinigungsmittel für harte Oberflächen, insbesondere Reinigungsmittel für Geschirr und unter diesen vorzugsweise solche für den Einsatz in maschinellen Reinigungsverfahren, die eine makrozyklische Verbindung L, 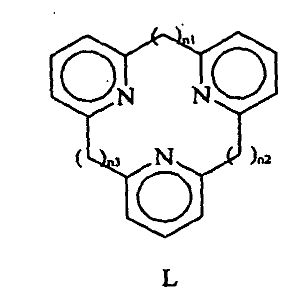 in der n1, n2 und n3 unabhängig voneinander 1 oder 2 sind, und Alkali perboratmonohydrat, Alkaliperborat tetrahydrat, Alkalipercarbonat rad oder peroxycarbonsäure, enthalten. Solche Mittel enthalten vorzugsweise auch ein Salz von Mangan, Eisen, Cobalt, Kupfer, Ruthenium oder Molybdän, oder sie werden in Gegenwart solcher Metallsalze, die separat zugesetzt werden oder gegebenenfalls aus dem verwendeten Wasser stammen, eingesetzt, damit sich aus diesen und dem Liganden L ein bleichkatalysierender Komplex bilden kann. Bleichkatalysierende Komplexe mit dem Liganden L können ihre Wirkung bei Anwesenheit von Luftsauerstoff entfalten.

Die erfindungsgemäße Verwendung besteht im wesentlichen darin, in Gegenwart einer mit gefärbten Anschmutzungen verunreinigten harten Oberfläche Bedingungen zu schaffen, unter denen ein Oxidationsmittel und der Bleichkatalysator gemäß Formel (I) miteinander reagieren können, mit dem Ziel, stärker oxidierend wirkende Folgeprodukte zu erhalten. Solche Bedingungen liegen insbesondere dann vor, wenn beide Reaktionspartner in wäßriger Lösung aufeinander treffen. Dies kann durch separate Zugabe der Persauerstoffverbindung und des Bleichkatalysators zu einer gegebenenfalls reinigungsmittelhaltigen Lösung geschehen. Besonders vorteilhaft wird das erfindungsgemäße Verfahren jedoch unter Verwendung eines erfindungsgemäßen Reinigungsmittels für harte Oberflächen das den Bleichkatalysator und gegebenenfalls ein persauerstoffhaltiges Oxidationsmittel enthält, durchgeführt. Die Persauerstoffverbindung kann auch separat, in Substanz oder als vorzugsweise wäßrige Lösung oder Suspension, zur Lösung zugegeben werden, wenn ein peroxidfreies Reinigungsmittel verwendet wird. Auf die Zugabe der Persauerstoffverbindung kann gewünschtenfalss auch verzichtet werden, wenn das Verfahren in Gegenwart von gasförmigem Sauerstoff, beispielsweise aus der Luft, durchgerührt wird.

Je nach Verwendungszweck können die Bedingungen weit variiert werden. So kommen neben rein wäßrigen Lösungen auch Mischungen aus Wasser und geeigneten organischen Lösungsmitteln als Reaktionsmedium in Frage. Die Einsatzmengen an Persauerstoffverbindungen werden vorzugsweise so gewählt, daß in den Lösungen von 10 ppm bis 10 % Aktivsauerstoff, insbesondere von 50 ppm bis 5 000 ppm Aktivsauerstoff vorhanden sind. Auch die verwendete Menge an Bleichkatalysator hängt vom Anwendungszweck ab. Je nach gewünschtem Aktivierungsgrad werden 0,00001 Mol bis 0,025 Mol, vorzugsweise 0,0001 Mol bis 0,02 Mol Katalysator pro Mol Persauerstoffverbindung verwendet, doch können in besonderen Fällen diese Grenzen auch über- oder unterschritten werden.

Ein weiterer Gegenstand der Erfindung ist ein Reinigungsmittel für harte Oberflächen, insbesondere für Geschirr, welches 0,001 Gew.-% bis 1 Gew.-%, insbesondere 0,005 Gew.-% bis 0,1 Gew.-% eines Bleichkatalysators gemäß Formel (I) und Alkalperborat monohydrat, Akaliperborattetrahydrat, Alkali percarbonat und/oder peroxycarbonsäure neben üblichen, mit dem Bleichkatalysator verträglichen Inhaltsstoffen enthält. Der Bleichkatalysator kann in im Prinzip bekannter Weise an Trägerstoffen adsorbiert und/oder in Hüllsubstanzen eingebettet sein.

Die Erfindung betrifft daneben noch entsprechende Verfahren zur Reinigung von harten Oberflächen, insbesondere von Geschirr, unter Einsatz eines Liganden L beziehungsweise eines Bleichkatalysators der Formel (I) oder

Die erfindungsgemäßen Reinigungsmittel gemäß den Ansprüchen 6 oder 8, die als pulver- oder tablettenförmige Feststoffe, homogene Lösungen oder Suspensionen vorliegen können, können außer dem erfindungsgemäß verwendeten Bleichkatalysator im Prinzip alle bekannten und in derartigen Mitteln üblichen Inhaltsstoffe enthalten. Die erfindungsgemäßen Mittel können insbesondere Buildersubstanzen, oberflächenaktive Tenside, wassermischbare organische Lösungsmittel, Enzyme, Sequestrierungsmittel, Elektrolyte, pH-Regulatoren und weitere Hilfsstoffe, wie Silberkorrosionsinhibitoren, Schaumregulatoren, zusätzliche Persauerstoff-Aktivatoren sowie Farb- und Duftstoffe enthalten. Für den Einsatz in maschinellen Geschirrspülverfahren vorgesehene erfindungsgemäße Mittel sind vorzugsweise sogenannte "3in1"-Produkte, die die herkömmlichen Mittel Reiniger, Klarspüler und Regeneriersalz in einem Mittel vereinen.

Ein erfindungsgemäßes Reinigungsmittel für harte Oberflächen kanne darüber hinaus abrasiv wirkende Bestandteile, insbesondere aus der Gruppe umfassend Quarzmehle, Holzmehle, Kunststoffmehle, Kreiden und Mikroglaskugeln sowie deren Gemische, enthalten. Abrasivstoffe sind in den erfindungsgemäßen Reinigungsmitteln vorzugsweise nicht über 20 Gew.%, insbesondere von 5 Gew.-% bis 15 Gew.-%, enthalten.

Ein weiterer Erfindungsgegenstand ist ein niederalkalisches Mittel zum maschinellen Reinigen von Geschirr, dessen 1-gewichtsprozentige Lösung einen pH-Wert von 8 bis 11,5, vorzugsweise 9 bis 10,5 aufweist, enthaltend 15 Gew.-% bis 60 Gew.%, insbesondere 30 Gew.-% bis 50 Gew.-% wasserlösliche Builderkomponente, 5 Gew.-% bis 25 Gew.-%, insbesondere 10 Gew.-% bis 15 Gew.-% Bleichmittel auf Sauerstoffbasis jeweils bezogen auf das gesamte Mittel, welches einen Bleichkatalysator gemäß Formel (I), insbesondere in Mengen von 0,005 Gew.-% bis 0,1 Gew.%, enthält. Eine besonders bevorzugte Ausführungsform der Erfindung ist ein maschinelles Geschirrspülmittel, das in Form einer Tablette, vorzugsweise in Form einer mehrphasigen Tablette, bei welcher Gehalt der einzelnen Phasen an Bleichkatalysator gemäß Formel (I) beziehungsweise Ligandmolekül L unterschiedlich ist, vorliegt. Ein unterschiedlicher Gehalt bedeutet dabei zum Beispiel, daß eine Phase der Tablette den gesamten oder zumindest den weitaus überwiegenden Teil des Bleichkatalysators gemäß Formel (I) enthält, und eine andere Phase den gesamten oder zumindest den weitaus überwiegenden Teil an Bleichmittel auf Sauerstoffbasis. Es ist allerdings auch möglich, den gesamten oder zumindest den weitaus überwiegenden Teil sowohl von Bleichmittel auf Sauerstoffbasis wie auch an Bleichkatalysator gemäß Formel (I) gemeinsam in eine Phase des tablettenförmigen Mittels einzuarbeiten, und in den anderern oder zumindest einer anderen Phase der Tablette den gesamten oder zumindest den weitaus überwiegenden Teil der bleichempfindlichen Wirkstoffe, beispielsweise Enzyme, einzusetzen. Anstelle des bereits vorgebildeten Komplexes der Formel (I) kann auch das Ligandmolekül L vorliegen, wenn zusätzlich ein Metallsalz vorhanden ist oder zutritt, so daß sich der Komplex der Formel (I) unter den Anwendungsbedingungen bilden kann.

Als wasserlösliche Builderkomponenten insbesondere in den niederalkalischen Reinigungsmitteln kommen prinzipiell alle in maschinellen Geschirreinigungsmitteln üblicherweise eingesetzten Builder in Frage, zum Beispiel polymere Alkaliphosphate, die in Form ihrer alkalischen neutralen oder sauren Natrium- oder Kaliumsalze vorliegen: können. Beispiele hierfür sind Tetranatriumdiphosphat, Dinatriumdihydrogendiphosphat, Pentanatriumtriphosphat, sogenanntes Natriumhexametaphosphat sowie die entsprechenden Kaliumsalze beziehungsweise Gemische aus Natrium- und Kaliumsalzen. Ihre Mengen können im Bereich von bis zu etwa 35 Gew.-%, bezogen auf das gesamte Mittel liegen; vorzugsweise sind die erfindungsgemäßen Mittel jedoch frei von solchen Phosphaten. Weitere mögliche wasserlösliche Builderkomponenten sind zum Beispiel organische Polymere nativen oder synthetischen Ursprungs, vor allem Polycarboxylate, die insbesondere in Hartwasserregionen als Co-Builder wirken. In Betracht kommen beispielsweise Polyacrylsäuren und Copolymere aus Maleinsäureanhydrid und Acrylsäure sowie die Natriumsalze dieser Polymersäuren. Handelsübliche Produkte sind zum Beispiel Sokalan® CP 5 und PA 30 der Firma BASF. Zu den als Co-Builder brauchbaren Polymeren nativen Ursprungs gehören beispielsweise oxidierte Stärke, wie zum Beispiel aus der internationalen Patentanmeldung WO 94/05762 bekannt, und Polyaminosäuren wie Polyglutaminsäure oder Polyasparaginsäure. Weitere mögliche Builderkomponenten sind natürlich vorkommende Hydroxycarbonsäuren wie zum Beispiel Mono-, Dihydroxybemsteinsäure, α-Hydroxypropionsäure und Gluconsäure. Zu den bevorzugten Builderkomponenten gehören die Salze der Citronensäure, insbesondere Natriumcitrat. Als Natriumcitrat kommen wasserfreies Trinatriumcitrat und vorzugsweise Trinatriumcitratdihydrat in Betracht. Trinatriumcitratdihydrat kann als fein- oder grobkristallines Pulver eingesetzt werden. In Abhängigkeit vom letztlich in den erfindungsgemäßen Mitteln eingestellten pH-Wert können auch die zu den genannten Co-Builder-Salzen korrespondierenden Säuren vorliegen.

Als Bleichmittel auf Sauerstoffbasis kommen in erster Linie Alkaliperboratmono- beziehungsweise -tetrahydrat und/oder Alkalipercarbonat in Betracht, wobei Natrium das bevorzugte Alkalimetall ist. Der Einsatz von Natriumpercarbonat hat insbesondere in Reinigungsmitteln für Geschirr Vorteile, da es sich besonders günstig auf das Korrosionsverhalten an Gläsern auswirkt. Das Bleichmittel auf Sauerstoffbasis ist deshalb vorzugsweise ein Alkalipercarbonat, insbesondere Natriumpercarbonat. Zusätzlich oder insbesondere alternativ können auch bekannte Peroxycarbonsäuren, zum Beispiel Dodecandipersäure oder Phthalimidopercarbonsäuren, die gegebenenfalls am Aromaten substituiert sein können, enthalten sein. Überdies kann auch der Zusatz geringer Mengen bekannter Bleichmittelstabilisatoren wie beispielsweise von Phosphonaten, Boraten beziehungsweise Metaboraten und Metasilikaten sowie Magnesiumsalzen wie Magnesiumsulfat zweckdienlich sein. Da Reinigungsmittel üblicherweise in Luftatmosphäre angewendet werden, können die Mittel auch frei von Bleichmitteln sein, da die Bleichkatalysatoren gemäß Formel (I) auch bereits in Gegenwart von Luftsauerstoff eine Wirkung zeigen.

Zusätzlich zu den Bleichkatalysatoren gemäß Formel (I) können weitere als bleichaktivierende Wirkstoffe bekannte Übergangsmetallsalze beziehungsweise -komplexe und/oder konventionelle Bleichaktivatoren, das heißt Verbindungen, die unter Perhydrolysebedingungen gegebenenfalls substituierte Perbenzoesäure und/oder Peroxocarbonsäuren mit 1 bis 10 C-Atomen, insbesondere 2 bis 4 C-Atomen ergeben, eingesetzt werden; dann ist jedoch die Gegenwart von Bleichmittel auf Persauerstoffbasis erforderlich. Geeignet sind die eingangs zitierten üblichen Bleichaktivatoren, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Glykolurile, insbesondere Tetraacetylglykoluril (TAGU), acylierte Triazinderivate, insbesondere 1,5-Diacetyl-2,4-dioxohexahydro-1,3,5-triazin (DADHT), acylierte Phenylsulfonate, insbesondere Nonanoyloxy- oder Isononanoyloxybenzolsulfonat, acylierte mehrwertige Alkohole, insbesondere Triacetin, Ethylenglykoldiacetat und 2,5-Di-acetoxy-2,5-dihydrofuran sowie acetyliertes Sorbit und Mannit, und acylierte Zuckerderivate, insbesondere Pentaacetylglukose (PAG), Pentaacetylfruktose, Tetraacetylxylose und Oetaacetyllactose sowie acetyliertes, gegebenenfalls N-alkyliertes Glucamin und Gluconolacton. Auch Kombinationen konventioneller Bleichaktivatoren können eingesetzt werden.

Vorzugsweise enthalten erfindungsgemäße maschinelle Geschirreinigungsmittel die üblichen Alkaliträger wie zum Beispiel Alkalisilikate, Alkalicarbonate und/oder Alkalihydrogencarbonate. Zu den üblicherweise eingesetzten Alkaliträgern zählen Carbonate, Hydrogencarbonate und Alkalisilikate mit einem Molverhältnis SiO₂/M₂O (M = Alkaliatom) von 1,5 : 1 bis 2,5 : 1. Alkalisilikate können dabei in Mengen von bis zu 30 Gew.-%, bezogen auf das gesamte Mittel, enthalten sein. Auf den. Einsatz der hoch alkalischen Metasilikate als Alkaliträger wird vorzugsweise ganz verzichtet. Das in den erfindungsgemäßen Mitteln bevorzugt eingesetzte Alkaliträgersystem ist ein Gemisch aus Carbonat und Hydrogencarbonat, vorzugsweise Natriumcarbonat und Hydrogencarbonat, das in einer Menge von bis zu 60 Gew.%, vorzugsweise 10 Gew.-% bis 40 Gew.-%, enthalten ist. Je nachdem, welcher pH-Wert letztendlich gewünscht wird, variiert das Verhältnis von eingesetztem Carbonat und eingesetztem Hydrogencarbonat, üblicherweise wird jedoch ein Überschuß an Natriumhydrogencarbonat eingesetzt, so daß das Gewichtsverhältnis zwischen Hydrogencarbonat und Carbonat im allgemeinen 1 : 1 bis 15 : 1 beträgt.

In einer weiteren bevorzugten Ausführungsform erfindungsgemäßer Mittel sind 20 Gew.-% bis 40 Gew.-% wasserlöslicher organischer Builder, insbesondere Alkalicitrat, 5 Gew.-% bis 15 Gew.-% Alkalicarbonat und 20 Gew.-% bis 40 Gew.-% Alkalidisilikat enthalten.

Den erfindungsgemäßen Mitteln können gegebenenfalls auch Tenside, insbesondere schwach schäumende nichtionische Tenside zugesetzt werden, die der besseren Ablösung fetthaltiger Anschmutzungen, als Netzmittel und gegebenfalls im Rahmen der Herstellung der Reinigungsmittel als Granulierhilfsmittel dienen. Ihre Menge kann bis zu 10 Gew.%, insbesondere bis zu 5 Gew.-% betragen und liegt vorzugsweise im Bereich von 0,5 Gew.-% bis 3 Gew.%. Üblicherweise werden insbesondere in Reinigungsmitteln für den Einsatz in maschinellen Geschirrspülverfahren extrem schaumarme Verbindungen eingesetzt. Hierzu zählen vorzugsweise C₁₂-C₁₈-Alkylpolyethylenglykol-polypropylenglykolether mit jeweils bei zu 8 Mol Ethylenoxid- und Propylenoxideinheiten im Molekül. Man kann aber auch andere bekannt schaumarme nichtionische Tenside verwenden, wie zum Beispiel C₁₂-C₁₈-Alkylpolyethylenglykol-polybutylenglykolether mit jeweils bis zu 8 Mol Ethylenoxid- und Butylenoxideinheiten im Molekül, endgruppenverschlossene Alkylpolyalkylenglykolmischether sowie die zwar schäumenden, aber ökologisch attraktiven C₈-C₁₄-Alkylpolyglucoside mit einem Polymerisierungsgrad von etwa 1 bis 4 (z. B. APG® 225 und APG® der Firma Henkel) und/oder C₁₂-C₁₄-Alkylpolyethylenglykole mit 3 bis 8 Ethylenoxideinheiten im Molekül. Ebenfalls geeignet sind Tenside aus der Familie der Glucamide wie zum Beispiel Alkyl-N-Methyl-Glucamide, in dienen der Alkylteil bevorzugt aus einem Fettalkohol mit der C-Kettenlänge C₆-C₁₄ stammt. Es ist teilweise vorteilhaft, wenn die beschriebenen Tenside als Gemische eingesetzt werden, zum Beispiel die Kombination Alkylpolyglykosid mit Fettalkoholethoxylaten oder Glucamid mit Alkylpolyglykosiden.

Obwohl Übergangsmetallkomplexe, insbesondere Mangankomplexe bekanntermaßen der Korrosion von Silber entgegenwirken können, werden die Verbindungen der Formel (I) in der Regel in Mengen eingesetzt, die zu gering sind, um einen Silberkorrosionsschutz zu bewirken, so daß in erfindungsgemäßen Reinigungsmitteln für Geschirr Silberkorrosionsinhibitoren noch zusätzlich eingesetzt werden können, deren Wirkung durch die Verbindungen gemäß Formel (I) verstärkt werden kann. Bevorzugte Silberkorrosionsschutzmittel sind organische Disulfide, zweiwertige Phenole, dreiwertige Phenole, gegebenenfalls substituiertes Benzotriazol, Mangan-, Titan-, Zirkonium-, Hafnium-, Vanadium-, Cobalt- oder Cersalze und/oder -komplexe, in denen die genannten Metalle in einer der Oxidationsstufen II, III, IV, V oder VI vorliegen.

Zusätzlich können die erfindungsgemäßen Mittel Enzyme wie Proteasen, Amylasen, Pullulanasen, Cutinasen und Lipasen enthalten, beispielsweise Proteasen wie BLAP®, Optimase®, Opticlean®, Maxacal®, Maxapem®, Esperase® und/oder Savinase®, Amylasen wie Termamyl®, Amylase-LT®, Maxamyl® und/oder Duramyl®, Lipasen wie Lipolase®, Lipomax®, Lumafast® und/oder Lipozym®. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind in den erfindungsgemäßen Reinigungsmitteln in Mengen von vorzugsweise nicht über 2 Gew.%, insbesondere von 0,1 Gew.-% bis 0,7 Gew.-%, enthalten.

Sofern die Reinigungsmittel bei der Anwendung zu stark schäumen, können ihnen noch bis zu 6 Gew.%, vorzugsweise etwa 0,5 Gew.-% bis 4 Gew.-% einer schaumdrilckenden Verbildung, vorzugsweise aus der Gruppe der Silikonöle, Gemische aus Silikonöl und hydrophobierter Kieselsäure, Paraffine, Parafin-Alkohol-Kombinationen, hydrophobierter Kieselsäure, der Bisfettsäureamide, und sonstiger weiterer bekannter im Handel erhältliche Entschäumer zugesetzt werden. Weitere fakultative Inhaltsstoffe in den erfindungsgemäßen Mitteln sind zum Beispiel Parfümöle.

Zu den in den erfindungsgemäßen Mitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Reinigungsmitteln vorzugsweise nicht über 20 Gew.%, insbesondere von 1 Gew.-% bis 15 Gew.-%, vorhanden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure oder Alkalihydrogensulfate, oder Basen, insbesondere Ammonium- oder Alkalihydroxide, enthalten. Derartige pH-Regulatoren sind in den erfindungsgemäßen Mitteln vorzugsweise nicht über 10 Gew.%, insbesondere von 0,5 Gew.-% bis 6 Gew.-%, enthalten.

Die Herstellung der erfindungsgemäßen festen Mittel bereitet keine Schwierigkeiten und kann in im Prinzip bekannter Weise, zum Beispiel durch Sprühtrocknen oder Granulation, erfolgen, wobei Persauerstoffverbindung und Bleichkatalysator gegebenenfalls später getrennt zugesetzt werden. Gegebenenfalls wird der Bleichkatalysator mit weiteren Rohstoffen und/oder Compounds vermischt und die Mischung anschließend zu Tabletten oder Phasen hiervon verpreßt.

Erfindungsgemäße Reinigungsmittel in Form wäßriger oder sonstige übliche Lösungsmittel enthaltender Lösungen werden besonders vorteilhaft durch einfaches Mischen der Inhaltsstoffe, die in Substanz oder als Lösung in einen automatischen Mischer gegeben werden können, hergestellt.

Die erfindungsgemäßen Mittel liegen vorzugsweise als pulverförmige, granulare oder tablettenförmige Präparate vor, die in an sich bekannter Weise, beispielsweise durch Mischen, Granulieren, Walzenkompaktieren und/oder durch Sprühtrocknung der thermisch belastbaren Komponenten und Zumischen der empfindlicheren Komponenten, zu denen insbesondere Enzyme, Bleichmittel und der Bleichkatalysator zu rechnen sind, hergestellt werden können.

Zur Herstellung von erfindungsgemäßen Reinigungsmitteln in Tablettenform geht man vorzugsweise derart vor, daß man alle Bestandteile beziehungsweise alle die für den gemeinsamen Einsatz in einer Phase der Tablette vorgesehenen Bestandteile in einem Mischer miteinander vermischt und das Gemisch beziehungsweise nacheinander die Gemische mittels herkömmlicher Tablettenpressen, beispielsweise Exzenterpressen oder Rundläuferpressen, mit Preßdrucken im Bereich von 200 10⁵ Pa bis 1 500 10⁵ Pa verpresst. Man erhält so problemlos bruchfeste und dennoch unter Anwendungsbedingungen ausreichend schnell lösliche Tabletten mit Biegefestigkeiten von normalerweise über 150 N. Vorzugsweise weist eine derart hergestellte Tablette ein Gewicht von 15 g bis 40 g, insbesondere von 20 g bis 30 g auf. Sie kann, beispielsweise bei einem Durchmesser von 35 mm bis 40 mm, rund sein oder jede andere gewünschte Form, beispielsweise rechteckig mit gegebenenfalls abgerundeten Kanten, aufweisen.

Die Herstellung erfindungsgemäßer Mittel in Form von nicht staubenden, lagerstabil rieselfähigen Pulvern und/oder Granulaten mit hohen Schüttdichten im Bereich von 800 bis 1000 g/l kann dadurch erfolgen, daß man in einer ersten Verfahrensteilstufe die BuilderKomponenten mit wenigstens einem Anteil flüssiger Mischungskomponenten unter Erhöhung der Schüttdichte dieses Vorgemisches vermischt und nachfolgend - gewünschtenfalls nach einer Zwischentrocknung - die weiteren Bestandteile des Mittels, darunter den Bleichkatalysator, mit dem so gewonnenen Vorgemisch vereinigt.

Erfindungsgemäße Mittel zur Reinigung von Geschirr können sowohl in Haushaltsgeschirrspülmaschinen wie in gewerblichen Spülmaschinen eingesetzt werden. Die Zugabe erfolgt von Hand oder mittels geeigneter Dosiervorrichtungen. Die Anwendungskonzentrationen in der Reinigungsflotte betragen in der Regel etwa 1 bis 8 g/l, vorzugsweise 2 bis 5 g/l.

Ein maschinelles Spülprogramm wird im Allgemeinen durch einige auf den Reinigungsgang folgende Zwischenspülgänge mit klarem Wasser und einem Klarspülgang mit einem gebräuchlichen Klarspülmittel ergänzt und beendet. Nach dem Trocknen erhält man beim Einsatz erfindungsgemäßer Mittel ein völlig sauberes und in hygienischer Hinsicht einwandfreies Geschirr.

## Patentansprüche

1. Verwendung von Übergangsmetall-Komplexen der Formel (I),
[MLₐX_{b}] cY (I)
in der
M für Mangan, Eisen, Cobalt, Kupfer, Ruthenium oder Molybdän,
L für den makrozyklischen Liganden 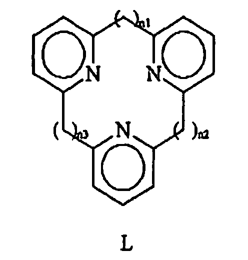 in dem n1, n2 und n3 unabhängig voneinander 1 oder 2 sind,
X für einen Neutral- oder Anionliganden,
Y für ein nicht komplexgebundenes Anion,
a für 1 oder 2 steht und
b und c unabhängig voneinander für Zahlen von 0 bis 6 stehen mit der Maßgabe, daß ihre Summe so zu wählen ist, daß sich in Abhängigkeit von der Ladung des Zentralatoms M und der Ladungen von X und Y Neutralität der Verbindung ergibt,
als Aktivatoren für insbesondere anorganische Persauerstoffverbindungen in Reinigungslösungen für harte Oberflächen.

2. Verwendung von Komplexen der Formel (I) als Aktivatoren für Persauerstoffverbindungen in Reinigungslösungen für Geschirr.

3. Verwendung nach Anspruch 1 oder 2, **dadurch gekennzeichnet, daß** das Übergangsmetall M in der Verbindung nach Formel (I) Mangan ist.

4. Verwendung einer makrozyklischen Verbindung L, 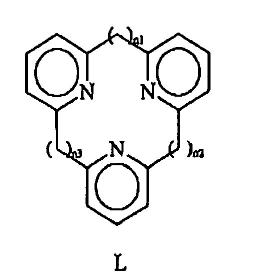 In der n1, n2 und n3 unabhängig voneinander 1 oder 2 sind. In Gegenwart eines Salzes von Mangan, Elsen, Cobalt, Kupfer, Ruthenium oder Molybdän, zur Verstärkung der Bleichleistung von insbesondere anorganischen Persauerstoffverbindungen in Reinigungslösungen für harte Oberflächen.

5. Verwendung nach einem der Ansprüche 1 bis 4, **dadurch gekennzeichnet, daß** die zu aktivierende Persauerstoffverbindung aus der Gruppe umfassend organische Persäuren, Wasserstoffperoxid, Perborat und Percarbonat sowie deren Gemische ausgewählt wird.

6. Reinigungsmittel für harte Oberflächen, enthaltend Alkaliperboratmonohydrat, Alkaliperborattetrahydrat, Alkalipercarbonat und/oder Peroxycarbonsäure, und eine makrozyklische Verbindung L, 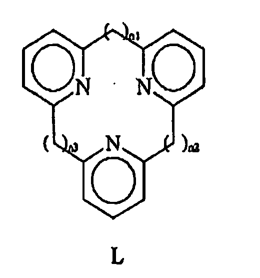 in der n1, n2 und n3 unabhängig voneinander 1 oder 2 sind,

7. Mittel nach Anspruch 6, **dadurch gekennzeichnet, dass** es auch ein Salz von Mangan, Elsen, Cobalt, Kupfer, Ruthenium oder Molybdän enthält.

8. Reinigungsmittel für harte Oberflächen, enthaltend Alkaliperboratmonohydrat, Alkaliperborattetrahydrat, Alkalipercarbonat und/oder Peroxycarbonsäure, und 0,001 Gew.-% bis 1 Gew.-%, insbesondere 0,005 Gew.-% bis 0,1 Gew.-% eines Bleichkatalysators gemäß Formel (I).

9. Mittel nach einem der Ansprüche 6 bis 8, **dadurch gekennzeichnet, dass** es sich um ein Reinigungsmittel für Geschirr, insbesondere für den Einsatz in maschinellen Reinigungsverfahren, handelt

10. Verfahren zur Reinigung von harten Oberflächen, insbesondere von Geschirr, unter Einsatz einer Verbindung der Formel (I).

11. Verfahren zur Reinigung von harten Oberflächen, insbesondere von Geschirr, unter Einsatz einer makrozyklischen Verbindung L, 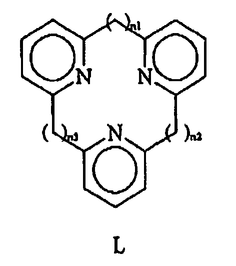 in der n1, n2 und n3 unabhängig voneinander 1 oder 2 sind, und eines Salzes von Mangen, Eisen, Cobalt, Kupfer, Ruthenium oder Molybdän, das gegebenenfalls aus dem verwendeten Wasser stammt.

## Claims

1. Use of transition-metal complexes of formula (I)
[MLₐX_{b}] cY (I)
in which
M denotes manganese, iron, cobalt, copper, ruthenium, or molybdenum,
L denotes the macrocyclic ligand 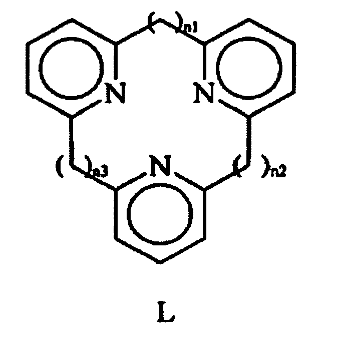 in which n1, n2, and n3, mutually independently, are 1 or 2,
X denotes a neutral or anionic ligand,
Y denotes a non-complex-bound anion,
a denotes 1 or 2, and
b and c mutually independently, denote numbers from 0 to 6, with the stipulation that their sum is to be selected to yield neutrality for the compound as a function of the charge of the central atom M and the charges of X and Y,
as activators for, in particular, inorganic peroxygen compounds in cleaning solutions for hard surfaces.

2. Use of complexes of formula (I) as activators for peroxygen compounds in cleaning solutions for tableware.

3. The use according to Claim 1 or 2, wherein the transition metal M in the compound according to formula (I) is manganese.

4. Use of a macrocyclic compound L 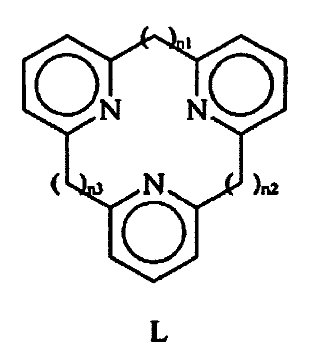 in which n1, n2, and n3, mutually independently, are 1 or 2, in the presence of a salt of manganese, iron, cobalt, copper, ruthenium, or molybdenum, to intensify the bleaching performance of, in particular, inorganic peroxygen compounds in cleaning solutions for hard surfaces.

5. The use according to one of Claims 1 to 4, wherein the peroxygen compound to be activated is selected from the group comprising organic peracids, hydrogen peroxide, perborate, and percarbonate, and mixtures thereof.

6. A cleaning agent for hard surfaces, containing alkali perborate monohydrate, alkaliperborate tetrahydrate, and/or peroxycarboxylic acid and a macrocyclic compound L 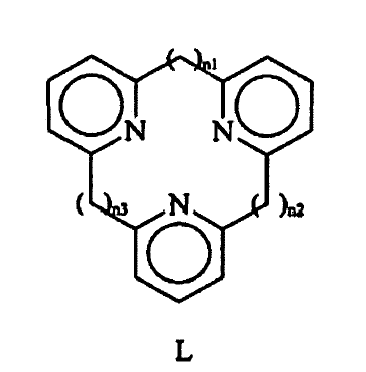 in which n1, n2, and n3, mutually independently, are 1 or 2.

7. The agent according to Claim 6, wherein it also contains a salt of manganese, iron, cobalt, copper, ruthenium, or molybdenum.

8. A cleaning agent for hard surfaces, containing alkali perborate monohydrate, alkaliperborate tetrahydrate, and/or peroxycarboxylic acid and 0.001 wt% to 1 wt%, in particular 0.005 wt% to 0.1 wt%, of a bleach catalyst according to formula (I).

9. The agent according to one of Claims 6 to 8, wherein it refers to a cleaning agent for tableware, in particular for use in automatic cleaning methods.

10. A method for cleaning hard surfaces, in particular tableware, using a compound of formula (I).

11. A method for cleaning hard surfaces, in particular tableware, using a macrocyclic compound L 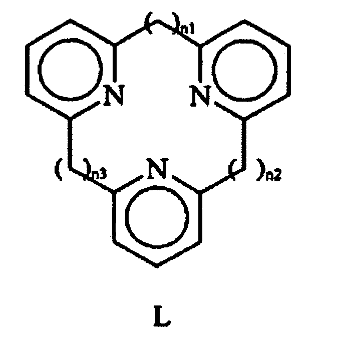 in which n1, n2, and n3, mutually independently, are 1 or 2, and using a salt of manganese, iron, cobalt, copper, ruthenium, or molybdenum, which salt derives if applicable from the water utilized.

## Revendications

1. Utilisation de complexes de métaux de transition répondant à la formule (I) :
[MLₐX_{b}] cY (I)
dans laquelle
M représente le manganèse, le fer, le cobalt, le cuivre, le ruthénium ou le molybdène ;
L représente le ligand macrocyclique 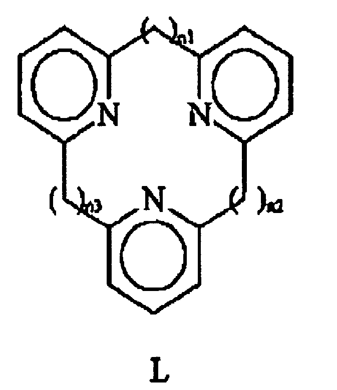 dans lequel n1, n2 et n3 sont égaux, indépendamment les uns des autres, à 1 ou 2 ;
X représente un ligand neutre ou anionique ;
Y représente un anion non lié au complexe ;
a est égal à 1 ou 2 ; et
b et c représentent, indépendamment l'un de l'autre des nombres de 0 à 6, avec cette mesure que leur somme doit être sélectionnée de telle sorte qu'en fonction de la charge de l'atome central M et des charges de X et Y, on obtient une neutralité du composé ;
comme activateurs pour des composés peroxygénés en particulier inorganiques dans des solutions de nettoyage pour des surfaces dures.

2. Utilisation de complexes répondant à la formule (I) comme activateurs pour des composés peroxygénés dans des solutions de nettoyage pour la vaisselle.

3. Utilisation selon la revendication 1 ou 2, **caractérisée en ce que** le métal de transition M dans le composé répondant à la formule (I) est du manganèse.

4. Utilisation d'un composé macrocyclique L, 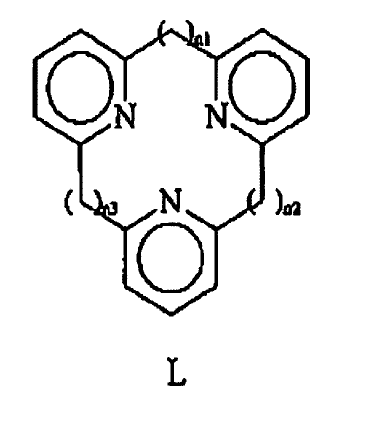 dans lequel n1, n2 et n3 sont égaux, indépendamment les uns des autres, à 1 ou 2, en présence d'un sel de manganèse, de fer, de cobalt, de cuivre, de ruthénium ou de molybdène, pour renforcer l'activité de blanchiment de composés peroxygénés en particulier inorganiques dans des solutions de nettoyage pour des surfaces dures.

5. Utilisation selon l'une quelconque des revendications 1 à 4, **caractérisée en ce que** le composé peroxygéné à activer est choisi parmi le groupe comprenant des peracides organiques, le peroxyde d'hydrogène, un perborate et un percarbonate ainsi que leurs mélanges.

6. Agent de nettoyage pour des surfaces dures, contenant un perborate de métal alcalin monohydraté, un perborate de métal alcalin tétrahydraté, un percarbonate de métal alcalin et/ou de l'acide peroxycarboxylique, et un composé macrocyclique L, 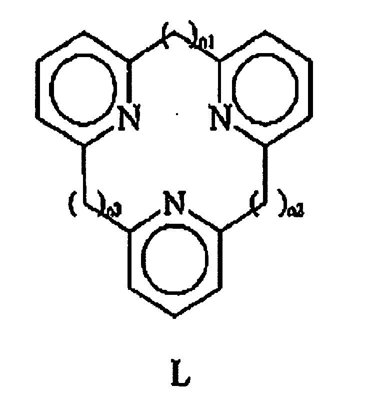 dans lequel n1, n2 et n3 sont égaux, indépendamment les uns des autres, à 1 ou 2.

7. Agent selon la revendication 6, **caractérisé en ce qu'**il contient également un sel de manganèse, de fer, de cobalt, de cuivre, de ruthénium ou de molybdène.

8. Agent de nettoyage pour des surfaces dures, contenant un perborate de métal alcalin monohydraté, un perborate de métal alcalin tétrahydraté, un percarbonate de métal alcalin et/ou de l'acide peroxycarboxylique, et, à concurrence de 0,001 % en poids à 1 % en poids, en particulier de 0,005 % en poids à 0,1 % en poids, un catalyseur du blanchiment répondant à la formule (I).

9. Agent selon l'une quelconque des revendications 6 à 8, **caractérisé en ce qu'**il s'agit d'un agent de nettoyage pour la vaisselle, en particulier pour une mise en oeuvre dans des procédés de nettoyage mécanique.

10. Procédé pour le nettoyage de surfaces dures, en particulier de la vaisselle, dans lequel on met en oeuvre un composé répondant à la formule (I).

11. Procédé pour le nettoyage de surfaces dures, en particulier de la vaisselle, dans lequel on met en oeuvre un composé macrocyclique L, 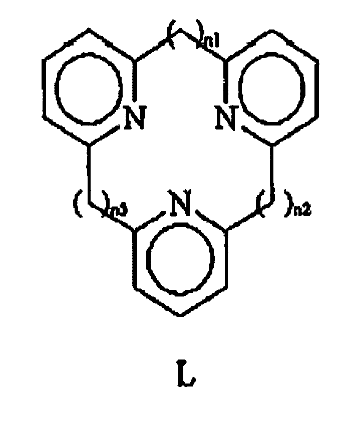 dans lequel n1, n2 et n3 sont égaux, indépendamment les uns des autres, à 1 ou 2, et d'un sel de manganèse, de fer, de cobalt, de cuivre, de ruthénium ou de molybdène qui provient, le cas échéant de l'eau utilisée.
